(19) Europäisches Patentamt
European Patent Office
Office européen des brevets

(11) EP 3 082 887 B1

(12) FASCICULE DE BREVET EUROPEEN

(45) Date de publication et mention
de la délivrance du brevet:
**29.08.2018 Bulletin 2018/35**

(21) Numéro de dépôt: **14830839.8**

(22) Date de dépôt: **19.12.2014**

(51) Int Cl.:
**A61L 26/00** *(2006.01)*      **A61F 13/02** *(2006.01)*

(86) Numéro de dépôt international:
**PCT/FR2014/053446**

(87) Numéro de publication internationale:
**WO 2015/092315 (25.06.2015 Gazette 2015/25)**

(54) **MATERIAU COMPOSITE DE REMPLISSAGE DES PLAIES CAVITAIRES**

VERBUNDWERKSTOFF ZUM FÜLLEN VON HOHLRAUMWUNDEN

COMPOSITE MATERIAL FOR FILLING CAVITY WOUNDS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **20.12.2013 FR 1363162**

(43) Date de publication de la demande:
**26.10.2016 Bulletin 2016/43**

(73) Titulaire: **URGO RECHERCHE INNOVATION ET
DEVELOPPEMENT
21300 Chenôve (FR)**

(72) Inventeur: **PERNOT, Jean-Marc
21000 Dijon (FR)**

(74) Mandataire: **Cabinet Plasseraud
66, rue de la Chaussée d'Antin
75440 Paris Cedex 09 (FR)**

(56) Documents cités:
**WO-A1-2009/071928      WO-A1-2009/071938
FR-A1- 2 783 412**

# EP 3 082 887 B1

**Description**

La présente invention a pour objet un matériau composite de remplissage des plaies (« wound packing » en terminologie anglo-saxone), notamment des plaies cavitaires, pouvant être utilisé dans les traitements topiques des plaies mettant en oeuvre des dispositifs à pression négative.

Ces dernières années, les traitements topiques des plaies mettant en oeuvre des dispositifs de thérapie par pression négative (TPN) se sont particulièrement développés dans le domaine de la cicatrisation des plaies du fait de leur capacité à accélérer la durée et la qualité de la cicatrisation.

Le principe de base des traitements TPN est de créer une cavité fermée sur la plaie au moyen d'un film d'étanchéité mince, souple, et collé à la peau du patient entourant la plaie. La cavité permet en outre d'admettre une extrémité d'un conduit d'aspiration, le conduit étant, par exemple, scellé sur le film d'étanchéité et raccordé, à son autre extrémité, à une pompe à vide capable de créer à l'intérieur de la cavité, une pression inférieure à la pression atmosphérique ambiante qui entoure la plaie. La dépression créée à l'intérieur de la cavité procure de nombreux effets thérapeutiques bénéfiques pour la cicatrisation, comme notamment une augmentation de la circulation sanguine et une granulation plus rapide des tissus. Différentes variantes de ces traitements TPN existent aujourd'hui.

Les traitements topiques des plaies mettant en oeuvre des dispositifs à pression négative permettent de traiter différents types de plaies, des plus petites lésions aux plus grandes plaies cavitaires ou aux brûlures quelle que soit leur taille. Ces blessures peuvent aussi être profondes et présenter, par conséquent, un volume important.

Il est nécessaire de contrôler la manière dont la plaie cicatrise. Idéalement, la lésion devrait cicatriser en profondeur au début, puis se refermer en rejoignant les bords de la plaie de manière uniforme. Il est en particulier souhaitable que la plaie ne se referme pas en surface avant que la cicatrisation profonde ne soit terminée, afin d'éviter la formation de cavités dans la chair qui deviendraient des sites favorables aux infections. Pour éviter la formation de ces cavités lors du traitement par TPN, la plaie est généralement comblée au moyen d'un matériau poreux mou ou compressible, et présentant des propriétés lui permettant de résister à la différence de pression créée à l'intérieur de la plaie par rapport à la pression atmosphérique ambiante. Le but du matériau est de maintenir les bords de la plaie suffisamment éloignés pour qu'ils ne puissent pas croître et se rejoindre pour former une cavité non souhaitée.

Le matériau doit également permettre de fournir des canaux d'écoulement des fluides afin, de permettre une aspiration efficace des exsudats hors de la plaie, en général dans un réceptacle de déchets, également appelé réservoir, associé au conduit aspirant.

Les matériaux mis en oeuvres dans les systèmes TPN sont à distinguer des pansements lamellaires multicouches classiques qui visent à protéger de manière superficielle les lésions de petite taille et sensiblement planes, et ne sont pas configurés pour être introduits à l'intérieur d'une plaie, mais simplement pour être positionnés à la surface de celle-ci, de manière à l'isoler de l'environnement extérieur le temps de la cicatrisation. En particulier, les pansements lamellaires ne forment pas des canaux d'écoulement de fluides pour permettre un drainage efficace des exsudats. En outre, les matériaux constituant ces pansements lamellaires, en particulier constituant la couche externe de ces pansements, présentent une certaine adhérence aux tissus, et n'ont pas vocation à être mis en contact avec la plaie. Une utilisation dans une plaie cavitaire entraînerait inévitablement un contact entre le matériau constituant la couche externe du pansement et les tissus de la plaie, ce qui générerait un arrachage tout à fait inacceptable des bourgeons tissulaires lors du retrait du pansement.

La plupart des systèmes TPN commercialisés aujourd'hui mettent en oeuvre un matériau à base de mousse ou de gaze comme matériau de remplissage de la plaie. Cependant, les matériaux poreux de ce type présentent l'inconvénient de favoriser la croissance tissulaire dans leurs pores, croissance tissulaire qui s'accroche au niveau desdits pores, ce qui, lors du retrait du matériau, peut endommager les tissus de granulation nouvellement formés et être douloureux pour le patient. En outre, de tels matériaux poreux peuvent également laisser les exsudats au contact de la plaie, provoquant une accumulation de bactéries menant jusqu'à l'infection.

Des développements ont ainsi été proposés afin d'envelopper les matériaux poreux type mousse ou gaze par des matériaux non poreux mais aussi non adhérents aux tissus cutanés au contact desquels ces derniers se trouvent appliqués. Les demandes internationales WO2009/071928 et WO2009/071938 de Smith and Nephew ont notamment proposé des matériaux composites de remplissage des plaies comprenant une enveloppe non poreuse, renfermant un matériau résilient de type mousse ou gaze. L'enveloppe peut être additionnellement enrobée ou trempée dans un gel non-adhérent tel qu'un hydrogel ou un gel de silicone. En outre, il est également connu de dispenser des matériaux à base de fibres gélifiantes au contact de la plaie et ce sous forme plane. Ces matériaux à base de fibres gélifiantes et qui présentent l'avantage d'être non adhérents sont bien connus de l'homme du métier, et sont décrits par exemple dans la demande de brevet WO2006/052839. A titre d'exemple de ces matériaux à base de fibres gélifiantes, on peut notamment citer les carboxyméthylcellulose (CMC) ou ses sels, les alginates ou encore l'acide hyaluronique. Ces matériaux peuvent tout à fait être constitutifs de l'enveloppe du matériau composite de remplissage des plaies. Toutefois, les matériaux proposés dans la demande WO2006/052839 présentent l'inconvénient de se dissocier ou se délaminer sous l'effet de la différence de pression exercée par le TPN. Leur utilisation comme matériau composite de remplissage risquerait alors de libérer des débris dans la plaie, qui, à terme, pourrait s'infecter et/ou perturber le bon déroulement des différentes étapes du mécanisme de cicatrisation.

Il existe donc un réel besoin dans la réalisation d'un matériau composite de remplissage des plaies, notamment cavitaires, présentant les propriétés souhaitées de compressibilité et de résilience ou de déformabilité tout en assurant l'écoulement des exsudats sans adhérer aux cellules de la plaie, ledit matériau composite présentant en outre la faculté de résister mécaniquement aux diverses sollicitations mécaniques telles que les cycles de pressions exercés par la TPN, sans se déstructurer, et dont la présence d'un support pour maintenir le matériau gélifiant, constitutif de l'enveloppe, sous une forme native le plus longtemps possible n'est pas nécessaire.

La présente invention a ainsi pour objet un matériau composite de remplissage de plaie comprenant une enveloppe renfermant un matériau ou un ensemble de matériaux formant des canaux d'écoulement des fluides, ladite enveloppe étant constituée d'un matériau interface autoporté formé d'une couche mince d'une composition comprenant une matrice hydrophobe et comportant des trous traversants, ladite matrice hydrophobe comprenant :

- pour 100 parties en poids d'un copolymère tribloc styrène-oléfine saturée-styrène présentant une viscosité comprise entre 0,2 et 2 Pa.s, telle que mesurée dans une solution à 10 % (masse/masse) dans le toluène ;

- de 400 à 1 220 parties en poids d'un plastifiant, de préférence une huile plastifiante ; et

- de 0 à 720 parties en poids de vaseline ;

étant en outre précisé que :

- la quantité totale de plastifiant et de vaseline est supérieure ou égale à 750 parties en poids ;

- la quantité de vaseline est comprise entre 400 et 720 parties en poids lorsque la quantité de plastifiant est comprise entre 1 000 et 1 220 parties en poids.

L'enveloppe du matériau composite selon l'invention est de préférence constituée d'un matériau interface autoporté, c'est-à-dire qu'il n'est pas nécessaire de lui associer un support pour en maintenir la forme.

On entend par matériau composite de remplissage de plaie au sens de la présente invention une structure de type coeur/écorce, le coeur étant constitué par un matériau ou un ensemble de matériaux formant des canaux d'écoulement de fluide, l'écorce, appelée également enveloppe, étant constituée d'un matériau interface autoporté, c'est-à-dire qu'il n'est pas nécessaire de lui associer un support pour en maintenir la forme.

Figures :

Les figures 1 A et B sont des schémas explicatifs de deux modes de réalisation d'un matériau de remplissage de plaie comprenant une enveloppe renfermant un matériau ou un ensemble de matériaux formant des canaux d'écoulement de fluide (l'écoulement des exsudats étant matérialisé, de manière purement illustrative, par des flèches sur les figures).

La figure 2 présente schématiquement un support de l'enveloppe du matériau composite selon l'invention, ledit tricot présentant des mailles trapézoïdales.

La figure 3 est une photographie du montage du dispositif mis en oeuvre pour réaliser le test de résistance mécanique mis en oeuvre dans les exemples selon l'invention.

La figure 4 est une photographie du dispositif monté mis en oeuvre pour réaliser le test de résistance mécanique mis en oeuvre dans les exemples selon l'invention.

La figure 5 est une photographie de l'enveloppe du matériau composite selon l'invention à l'état hydraté, après « 5 cycles » de test de résistance mécanique.

Matrice hydrophobe

Dans le cadre de la présente invention, l'enveloppe est constituée d'un matériau interface autoporté formé d'une couche mince d'une composition comprenant une matrice hydrophobe.

Selon une caractéristique particulière, la matrice hydrophobe précitée comprend en outre des particules d'hydrocolloïdes en une quantité inférieure ou égale à 25 % en poids, rapportée au poids total de ladite matrice hydrophobe.

Selon un mode particulier de réalisation de l'invention, la matrice hydrophobe précitée est généralement constituée d'un élastomère de type copolymère tribloc styrène-oléfine saturée-styrène, d'un plastifiant et éventuellement de vaseline.

Cette matrice comporte, de préférence comme seul élastomère, un copolymère tribloc séquencé comportant deux blocs terminaux thermoplastiques styrène et une séquence centrale élastomérique constituée d'une polyoléfine saturée.

Cette polyoléfine saturée peut être de type poly(éthylène-butylène) ou poly(éthylène-propylène).

Les copolymères triblocs à séquence centrale saturée sont bien connus de l'homme de l'art. Ces composés présentent des propriétés variées et se différencient, en particulier, par leur viscosité.

Il a été découvert que la réalisation d'un matériau autoporté au sens de l'invention est subordonnée au choix d'un copolymère de viscosité spécifique.

Ainsi, dans le cadre de la présente invention, l'élastomère sera spécifiquement choisi parmi les copolymères présentant un haut poids moléculaire, caractérisés par une viscosité comprise entre 0,2 et 2 Pa.s telle que mesurée dans une solution à 10 % (masse/masse) dans le toluène.

Des copolymères triblocs à séquence centrale saturée répondant à cette propriété sont par exemple commercialisés :

- par la société KRATON POLYMERS sous la dénomination KRATON® G1651 ou KRATON® G1654 pour les copolymères séquencés poly(styrène-éthylène-butylène-styrène)(en abrégé SEBS) ;

- par la société KURARAY sous la dénomination SEPTON® S2006 pour les copolymères séquencés poly(styrène-éthylène-propylène- styrène) (en abrégé SEPS) et SEPTON® S8006 pour les copolymères séquencés SEBS.

Dans le cadre de la présente invention, on préférera les copolymères triblocs SEBS ou SEPS ayant une teneur en styrène comprise entre 25 et 45 % en poids, rapporté au poids total dudit copolymère.

D'une façon préférée, on utilisera les produits commercialisés par la société KRATON POLYMERS sous les dénominations KRATON® G1651 et KRATON® G1654.

Pour obtenir un matériau interface autoporté, non seulement la nature de l'élastomère utilisé doit être spécifiquement choisie, mais en outre, cet élastomère devra être utilisé dans des proportions relatives spécifiques au sein de la matrice hydrophobe, comme on le verra par la suite.

Le plastifiant utilisé pour la réalisation de la matrice hydrophobe est destiné à améliorer les propriétés d'étirement, de souplesse, d'extrudabilité et de mise en oeuvre de l'élastomère précité.

De préférence, ce plastifiant sera constitué d'un liquide ou d'un mélange de liquides compatible avec la séquence centrale polyoléfine saturée de l'élastomère utilisé.

Parmi les plastifiants susceptibles d'être avantageusement utilisés, on peut citer en particulier les huiles plastifiantes ou encore les produits de synthèse à base de mélanges liquides d'hydrocarbures saturés comme par exemple les produits commercialisés par la société TOTAL sous la dénomination GEMSEAL® et en particulier le produit GEMSEAL® 60 qui est un mélange isoparaffinique issu d'une coupe pétrolière totalement hydrogénée.

Dans le cadre de la présente invention, on préférera utiliser des huiles plastifiantes, de préférence des huiles minérales plastifiantes et en particulier des huiles minérales formées de composés de nature paraffinique ou naphténique ou de leurs mélanges dans des proportions variables.

Des huiles minérales plastifiantes particulièrement préférées sont formées de mélanges de composés de nature paraffinique et naphténique, et en particulier de tels mélanges dans lesquels la proportion de composés de nature paraffinique est majoritaire.

A titre d'exemple d'huile minérale plastifiante commerciale, on peut citer les produits commercialisés par la société SHELL sous la dénomination ONDINA®.

Parmi ces produits, d'excellents résultats ont été obtenus avec les huiles commercialisées sous les dénominations ONDINA® 917 ou ONDINA® 919.

Comme indiqué précédemment, la quantité de plastifiant présente au sein de la matrice hydrophobe doit être spécifiquement choisie en fonction de la quantité d'élastomère.

Ainsi, pour 100 parties en poids d'élastomère, la matrice hydrophobe contiendra de 400 à 1 220 parties en poids, et de préférence de 600 à 900 parties en poids, de plastifiant, de préférence d'une huile plastifiante.

Pour augmenter le caractère et le toucher gras du mélange constitué par l'élastomère et le plastifiant, il peut être avantageux, dans le cadre de la présente invention, d'ajouter à ce mélange une quantité prédéterminée de vaseline.

Il s'agira de préférence d'une vaseline conforme à la Pharmacopée Française disponible commercialement.

La quantité de vaseline au sein de la matrice hydrophobe peut être généralement comprise entre 0 et 720 parties en poids, et de préférence de 150 à 450 parties en poids, pour 100 parties en poids d'élastomère.

Cependant, pour obtenir un matériau autoporté convenant à l'enveloppe selon l'invention, il a été constaté que les deux conditions suivantes doivent être nécessairement respectées :

- la quantité totale de plastifiant et de vaseline doit être supérieure ou égale à 750 parties en poids, pour 100 parties en poids d'élastomère ;

- la quantité de vaseline doit être comprise entre 400 et 720 parties en poids lorsque la quantité de plastifiant est comprise entre 1000 et 1220 parties en poids.

La matrice hydrophobe qui vient d'être décrite constitue l'élément essentiel des compositions permettant de réaliser l'enveloppe autoportée du matériau composite de remplissage conforme à l'invention.

L'enveloppe du matériau composite de remplissage de plaie selon la présente invention est constituée d'un matériau autoporté formé d'une couche mince d'une composition comprenant une matrice hydrophobe et comportant des trous traversant.

A cet effet, ces compositions comprenant une matrice hydrophobe seront formées en couche mince avec des trous traversant, disposés de préférence de façon répartie dans ladite couche.

Les trous traversant peuvent être réalisés par perforation ou poinçonnage d'une composition préalablement formée en couche mince, seule ou associée à un support provisoire ou à un film protecteur, ou bien encore par une enduction tramée sur un support provisoire. Pour plus de détails, on pourra se référer à la demande de brevet FR 2 936 158.

Alternativement, l'enveloppe du matériau composite de remplissage de plaie conforme à l'invention peut être fabriquée par coulage à chaud d'une composition comprenant la matrice hydrophobe telle que décrite précédemment sur une plaque gravée avec le motif retenu pour former des trous traversant, suivi d'un refroidissement et d'un démoulage.

D'une façon générale, l'enveloppe du matériau composite de remplissage de plaie conforme à l'invention présentera une épaisseur comprise entre 0,4 mm et 2 mm, de préférence entre 0,5 mm et 1 mm, de préférence encore de l'ordre de 0,6 mm.

Les trous traversant peuvent être de géométrie quelconque et présenteront par exemple une section transversale circulaire, rectangulaire, trapézoïdale ou carrée.

Leur surface est généralement comprise entre 0,25 et 5 mm$^2$.

Ces trous présentent notamment un diamètre moyen compris entre 0,5 et 2 mm, de préférence de l'ordre de 1 mm, lorsque leur section transversale est circulaire.

Ces trous sont, de préférence, répartis de façon régulière, avec une densité telle que la surface totale des trous représente entre 20 et 70 %, et de préférence entre 30 et 50 % de la surface totale de l'enveloppe.

Selon un mode de réalisation préféré, l'enveloppe en matériau autoporté selon l'invention se présente sous la forme d'un filet aéré (ou grille), de préférence de maille carrée, présentant :

- une épaisseur du filet comprise entre 0,4 et 2 mm, de préférence entre 0,5 mm et 1 mm ;

- une « largeur de fil » (largeur de l'espace entre deux trous consécutifs) comprise entre 1 et 10 mm;

- un grammage compris entre 200 et 1700 g/m$^2$, et de préférence compris entre 300 et 800 g/m$^2$.

Selon un mode de réalisation particulièrement préféré de l'invention, un tel matériau autoporté se présentera sous la forme d'un filet aéré à maille carrée présentant :

- une épaisseur du filet de 600 microns environ ;

- une largeur de fil de l'ordre de 2 mm ;

- un grammage de l'ordre de 450 g/m$^2$.

Selon un mode de réalisation alternatif, l'enveloppe est constituée d'un matériau interface autoporté formé d'une couche mince d'une composition comprenant une matrice hydrophobe pouvant éventuellement être réalisée à partir d'une composition comprenant une matrice hydrophobe comportant A parties d'un élastomère tribloc, B parties d'huile de plastification, C parties de polyéthylène, et un hydrocolloïde dispersé dans une proportion comprise entre 5 et 15 % du poids total de la matrice hydrophobe, avec les relations suivantes

$$A = 100$$

$$300 < B < 500$$

$$10 < C < 50$$

$$9\% < C/(A+C) < 33,5\%$$

$$1,5\% < C/(A + B + C) < 9\%$$

$$83\% > B/(A + B) > 75\%$$

Ladite matrice hydrophobe possède des trous traversant, que l'on peut comparer à des ouvertures permettant le passage des exsudats de la plaie.

Ladite matrice hydrophobe possède une épaisseur comprise entre 200 $\mu$m et 600 $\mu$m, ou mieux entre 300 $\mu$m et 500 $\mu$m, de préférence autour de 400 $\mu$m.

L'élastomère thermoplastique de synthèse, hydrophobe, de type tribloc, est avantageusement un élastomère de type SIS formé par copolymérisation de blocs de type polystyrène (par exemple 15%) avec des blocs de type isoprène. L'élastomère a de préférence un poids moléculaire moyen ou élevé et son taux de diblocs est par exemple de 18 %.

Le polyéthylène est apporté sous forme de granulés. Sa viscosité Brookfield se situe autour de 450 cps à 140°C et sa température de fusion comprise entre 92°C et 122°C.

Le plastifiant de la composition est l'huile, notamment une huile minérale ou végétale compatible avec les autres éléments de la composition et tolérée par la peau. On utilise de préférence une huile de paraffine de faible viscosité à base de composés paraffiniques et naphténiques ou des mélanges d'huile de paraffine.

La composition plastifiée est extensible et « élastique » et présente une certaine hystérésis avec un retour élastique d'au moins 3-5% pour un allongement de 20%.

Pour rendre plus anti-adhérent et pour maintenir au niveau de l'interface un environnement humide propice à la cicatrisation, on ajoute un hydrocolloïde en dispersion dans la matrice constituée des éléments ci-dessus définis. On pourra se référer au document précité EP 1 143 895 qui définit la nature et les conditions d'addition d'un hydrocolloïde convenant également à la présente invention. En pratique, on utilisera de préférence de la carboxyméthylcellulose de sodium (NaCMC) dans des proportions de 5% à 15% de la matrice hydrophobe.

Naturellement, la composition peut comprendre également divers produits antioxydants ou stabilisants, ainsi que des principes actifs qui ajouteront un effet particulier à la composition. Ces produits sont connus de l'homme du métier.

La fabrication de l'interface conforme à l'invention commence avec le malaxage à chaud et sans solvant selon la technique dite « hotmelt » (à une température d'environ 150°C) du polymère tribloc, du composant huileux, et du polyéthylène afin d'obtenir un mélange homogène, dans lequel on incorpore la CMC, à une température légèrement inférieure, par exemple, de 130°C-135°C.

Le produit du mélange est ensuite déposé en enduction d'une épaisseur choisie sur un support.

Le support peut être un support continu ou discontinu, il s'agit d'un support intermédiaire servant au transport et au stockage de l'interface jusqu'à son utilisation sous forme d'enveloppe pour matériau composite de remplissage d'une plaie. Le support peut être de toute nature du moment qu'il présente une surface non-adhésive permettant la séparation facile de l'interface et du support. Ledit support est avantageusement un support souple, tel qu'une feuille (film, pellicule) siliconée, par exemple une feuille de polyester silicone. On ajoute soit quasi simultanément soit lors d'une étape ultérieure une autre feuille siliconée au-dessus de l'interface de sorte que celle-ci est protégée sur ses deux faces. On pourra se référer pour une technique d'enduction entre deux supports à la figure page 153 de l'ouvrage « Coating and Laminating », Herbert Weiss, Converting Technology Machine, 1977.

Selon un aspect primordial de l'invention, le procédé prévoit la formation de trous dans l'interface enduite. Selon un premier mode de réalisation, les trous sont pratiqués par une méthode de perforation (par exemple par poinçon et matrice) de l'enduction alors qu'elle est déjà déposée sur son support souple, et de préférence alors qu'elle est protégée par ses deux feuilles souples de support et de protection.

Selon un second mode de réalisation, les trous sont formés dans l'enduction au moment même où celle-ci est déposée sur son support, par exemple par une machine commercialisée par Cavitec sous la référence Cavimel-TSM ou par Nordson sous la référence « system REA », ce procédé laissant automatiquement l'enduction sous forme d'une trame. Le principe est de déposer au moyen d'une buse le produit sur un cylindre tramé ou gravé qui ensuite transfère l'enduction tramée sur son support. Un tel procédé est par exemple connu par le documentWO/011352.

Les trous présentent un diamètre moyen de 1 à 4 mm, par exemple sous forme de trous ronds de 3 mm avec un pas de 8 mm.

Ladite matrice hydrophobe présente un pouvoir happant exclusif d'une adhésivité notable au sens mesurable du terme, l'interface de l'invention étant précisément non collante et ne provoquant aucun arrachement de tissu humain ou de bourgeon de cicatrisation lors de son enlèvement.

Selon un second mode de réalisation alternatif, une matrice hydrophobe élastomérique non absorbante convient tout particulièrement à la mise en oeuvre d'un matériau de remplissage de plaie cavitaire, objet de ladite invention.

Cette matrice se trouve décrite au sein des brevets Lohmann & Rauscher EP 2 524 706 et EP 2 524 705, dont les contenus se trouvent entièrement incorporés par référence à la présente description.

Selon ce second mode de réalisation alternatif, la matrice élastomérique hydrophobe peut comprendre au moins un élastomère tribloc synthétique de formule générale A-B-A, le bloc terminal A pouvant être de type polystyrène et le bloc central B pouvant être de type polyoléfine saturée.

Pour obtenir une matrice élastomérique hydrophobe selon ce second mode de réalisation alternatif, on utilise des élastomères tribloc de type polystyrène-b-poly(éthylène-butylène)-b-polystyrène (S-EB-S), ou polystyrène-b-poly(éthylène-propylène)-b-polystyrène (S-EP-S), ou polystyrène-b-poly(éthylène-éthylène/propylène)-b-polystyrène (S-EEP-S) de poids moléculaire élevé et ultra-élevé.

De manière avantageuse, on utilise des élastomères hydratés polystyrène-b-poly(éthylène-butylène)-b-polystyrène (S-EB-S, par exemple le Kraton G 1651). Il peut être également utilisé des élastomères hydratés polystyrène-b-poly(éthylène-éthylène/propylène)-b-polystyrène (S-EEP-S, tels que les Septon 4033, 4055, 4077 ou 4099).

Pour obtenir une matrice élastomérique hydrophobe selon ce second mode de réalisation alternatif, on choisit de préférence un mélange d'au moins deux élastomères tribloc de type S-EB-S ou S-EP-S, en particulier de type S-EEP-S, l'un des deux élastomères présentant un poids moléculaire élevé, c'est-à-dire un poids moléculaire supérieur à 150 000 Daltons et inférieur à 300 000 Daltons tel que mesuré par chromatographie d'exclusion stérique, et l'autre élastomère présentant un poids moléculaire ultra élevé, c'est-à-dire un poids moléculaire supérieur à 300 000 Daltons et inférieur à 600 000 Daltons tel que mesuré par chromatographie d'exclusion stérique et possédant chacun une viscosité Brookfield d'au moins 5000 mPas (mesuré dans une solution à 10% de toluène à 30°C).

La matrice hydrophobe élastomère peut comprendre entre 1,5 et 10% en poids d'élastomères, de préférence entre 2 et 3% et de façon encore plus préférée 2,6%.

Ainsi, une matrice comprenant une association d'un élastomère de la marque Kraton tel que le Kraton G 1651 ou d'un autre élastomère de la marque Kraton de la série G possédant la propriété de poids moléculaire souhaitée, à un élastomère de la marque Septon, tel que l'élastomère Septon 4055, l'élastomère Septon 4077 ou l'élastomère Septon 4099, est envisagée par ce mode de réalisation.

D'une autre manière avantageuse, la matrice hydrophobe élastomérique comprend deux élastomères de poids moléculaire ultra-élevé, c'est-à-dire de poids moléculaire supérieur à 300 000 Daltons et inférieur à 600 000 Daltons tel que mesuré par chromatographie d'exclusion stérique.

Ainsi, une matrice comprenant une association d'un élastomère de la marque Septon tel que l'élastomère Septon 4055, à un autre élastomère de la marque Septon tel que le Septon 4077 ou bien l'élastomère Septon 4099 est envisagée par ce mode de réalisation. Une association d'un élastomère Septon 4077 à un élastomère Septon 4099 est également envisagée.

Il est à noter que, au sein de la matrice hydrophobe élastomérique, la part totale de l'élastomère possédant le plus haut poids moléculaire est supérieure à 5%, de préférence supérieure à 10%, tout en étant inférieure à 50% de la part totale d'élastomères introduits dans la matrice.

Outre le (ou les) élastomère(s) tribloc(s), la matrice hydrophobe polymérique comprend également au moins un plastifiant huileux, constituant de préférence la majorité de la matrice hydrophobe élastomérique. Le plastifiant huileux peut notamment être constitué par une huile plastifiante et/ou de la vaseline. Du fait de la combinaison d'une forte proportion de plastifiant huileux avec une très faible proportion d'élastomères triblocs, la matrice se comporte comme un corps gras pur, de sorte qu'elle présente une très bonne compatibilité avec les tissus et de très bonnes propriétés de non adhérence à la plaie.

Parmi les plastifiants huileux convenant à la constitution de la matrice hydrophobe selon ce second mode de réalisation, c'est-à-dire permettant la plastification de l'élastomère, on peut mentionner les huiles de paraffine, les huiles blanches médicales, les huiles minérales, la vaseline, les paraffine de baume, les huiles de silicone, les cires ou encore leurs mélanges.

De préférence, le plastifiant sera constitué d'un liquide ou d'un mélange de liquides compatible(s) avec la séquence centrale polyoléfine saturée de l'élastomère utilisé.

Parmi les plastifiants susceptibles d'être avantageusement utilisés, on peut citer en particulier les huiles plastifiantes ou encore les produits de synthèse à base de mélanges liquides d'hydrocarbures saturés comme par exemple les produits commercialisés par la société TOTAL sous la dénomination GEMSEAL® et en particulier le produit GEMSEAL® 60 qui est un mélange isoparaffinique issu d'une coupe pétrolière totalement hydrogénée.

Dans le cadre de la présente invention, on préférera utiliser des huiles plastifiantes, de préférence des huiles minérales plastifiantes et en particulier des huiles minérales formées de composés de nature paraffinique ou naphténique ou de leurs mélanges dans des proportions variables.

Des huiles minérales plastifiantes particulièrement préférées sont formées de mélanges de composés de nature paraffinique et naphténique, et en particulier de tels mélanges dans lesquels la proportion de composés de nature paraffinique est majoritaire.

A titre d'exemple d'huile minérale plastifiante commerciale, on peut citer les produits commercialisés par la société SHELL sous la dénomination ONDINA®.

Parmi ces produits, d'excellents résultats ont été obtenus avec les huiles commercialisées sous les dénominations ONDINA® 917 ou ONDINA® 919.

La matrice peut également contenir des antioxydants. Comme antioxydants adaptés, on peut citer les antioxydants au souffre, par exemple le dibutyldithiocarbamate de zinc commercialisé sous la désignation PERKACIT ZDBC par la société Akzo Nobel Chemicals, et/ou les antioxydants au phénol, par exemple les produits commercialisés sous la dénomination IRGANOX® 1010, IRGANOX® 565 et IRGANOX® 1035 par la société BASF.

Dans le cadre de la présente invention, le composé IRGANOX®1010 est préféré.

La matrice élastomérique hydrophobe selon ce second mode de réalisation alternatif peut comprendre en outre un additif choisi parmi le groupe constitué des agents stabilisateurs, des auxiliaires d'extrusion, des charges de remplissage, des pigments, des colorants, des agents de réticulation, des agents anti-odorant, des agents d'adhérence, des agents de compatibilité et leurs combinaisons.

En outre ladite matrice peut comprendre au moins un type de particules organiques ou inorganiques hydrophiles fixant l'eau tel que les hydrocolloïdes ou les polymères superabsorbants. Parmi ceux-ci on peut citer la carboxymethylcellulose, les alginates, les xanthanes, les gélatines, les pectines, ainsi que leurs dérivés.

Un agent actif peut également être rajouté au sein de cette matrice. De façon préférée un biguanide, tel que la metformine ou le polyhexaméthylène-biguanide (PHMB), ou encore le stéarate de PHMB sera ajouté à ladite matrice.

Il est possible de définir cette matrice comme étant une matrice hydrophobe élastomérique non absorbante.

Selon ce second mode de réalisation alternatif, on entend par matrice élastomérique hydrophobe « non absorbante», toute matrice élastomérique hydrophobe telle que décrite précédemment et qui absorbe moins de 35% d'une solution saline (solution de NaCl à 0.8%) en 24h par rapport au poids sec de la matrice.

Selon une variante de ce mode de réalisation, ladite matrice peut être utilisée seule en guise d'enveloppe ou peut venir enduire un support tel que défini par la suite pour constituer ladite enveloppe.

Support de l'enveloppe

Selon l'une des caractéristiques de l'invention et, sous réserve de ne pas altérer les bonnes propriétés de cohésion du matériau composite selon l'invention, l'enveloppe constituée d'un matériau interface autoporté peut comprendre un support formé d'un tissu de fils en matériau flexible et très peu extensible et non élastique.

Ce support se présente sous forme d'un tissu à mailles larges ouvertes et peut être obtenu par des procédés de tissage ou de tricotage permettant de former des mailles ouvertes de taille régulière, carrées ou polygonales. Dans le cas d'un tissage, les mailles peuvent être fixées au moyen de fils de tour afin d'obtenir une bonne stabilité dimensionnelle. La dimension des mailles est telle que la surface unitaire des ouvertures est de l'ordre de 0,5 à 10 mm$^2$, préférentiellement 0,5 à 3 mm$^2$, le taux d'ouverture du tissu (rapport de la surface ouverte sur la surface totale) étant de l'ordre de 50 à 90 %. Le fil utilisé pour fabriquer le tissu est préférentiellement un fil continu à filaments, très peu extensible et non élastique, l'extensibilité ou l'allongement étant inférieure à 35 %. Par fil continu à filaments, on entend un fil formé de un ou plusieurs filaments longs retords ; le choix de filaments longs permet d'éviter les fibres courtes qui risquent de se détacher du support et venir se disperser près de la surface de contact avec la plaie.

Pour la même raison, le matériau constitutif des fils est de préférence de type hydrophobe, de nature artificielle ou synthétique ; ces constituants, comme par exemple les polyesters, les polyamides, les acétates de cellulose permettent d'obtenir des filaments longs et des fils présentant beaucoup moins de fibrilles que les fils obtenus à partir de fibres courtes par exemple. Le choix de certains matériaux synthétiques tels que des polyesters donne également la possibilité de thermofixer la structure à mailles larges du support. Le tissu à mailles larges est préférentiellement réalisé avec des fils de même nature, mais on peut utiliser aussi des tissus fabriqués par exemple avec des fils de chaîne et des fils de trame qui seraient de nature différente. Enfin, un autre avantage des matériaux très peu extensibles et non élastiques, tels que les polyesters, est une mise en oeuvre plus facile pendant le procédé de recouvrement des fils du tissu par le matériau constituant l'enveloppe.

Selon un mode préféré de réalisation de l'invention, on applique le matériau interface autoporté sur le tissu à mailles larges de façon à enrober les fils du tissu en laissant une majorité des mailles non obstruée.

Comme indiqué précédemment, on utilise un support tissé ou tricoté présentant des mailles larges rectangulaires, carrées ou polygonales dont l'ouverture correspond sensiblement à 4 à 20 mailles par cm, le tissu ayant un taux d'ouverture (rapport des surfaces ouvertes sur la surface totale) de 50 à 90 %. Le fil utilisé pour obtenir le support est de préférence un fil continu à filaments, et on choisit, pour réaliser les exemples préférés de l'invention, des fils en matériau artificiel ou synthétique, à caractère hydrophobe et d'extensibilité inférieure à 35 %.

La nature du fil est, par exemple, un polyester de type polyéthylène téréphtalate, un polyamide ou un acétate de cellulose ; on utilise de préférence un tissu à mailles larges thermofixées en fils continus de polyester (Tergal ou polyéthylène téréphtalate), par exemple des tissus commercialisés sous le nom de marquisette, grammant environ 30 à 80 g/m$^2$. Ces tissus pratiquement non extensibles dans les directions chaîne ou trame présentent l'avantage de se travailler plus facilement que les tissus élastiques et on obtient un enrobage plus régulier des fils.

Ce type de support formé d'un tissu de fils en matériau flexible et très peu extensible et non élastique est notamment décrit dans la demande de brevet WO 00/16725.

Alternativement, le tissu constituant le support de l'enveloppe peut être un tricot thermofixé avec fils tramés, lesdits fils étant des fils continus à filaments non élastiques, qui présente dans le sens transversal une extensibilité mesurée selon la norme NF EN 13726-4 comprise entre 0,01 et 0,5 N/cm, de préférence comprise entre 0,05 à 0,3 N/cm, et de préférence encore entre 0,08 à 0,15 N/cm.

Selon une version préférée de la présente invention, ce tricot présentera dans le sens longitudinal une extensibilité mesurée selon la même norme comprise entre 15 et 30 N/cm et de préférence entre 20 et 25 N/cm. Une telle extensibilité dans le sens longitudinal permet en effet un enrobage plus facile et plus régulier du tricot par le matériau interface autoporté du point de vue industriel.

De façon générale, ce tricot est réalisé avec fils tramés, et sera notamment fabriqué selon la technologie dite « mailles jetées ».

De plus, ce tricot est thermofixé. Cette thermofixation permet de stabiliser sur le plan dimensionnel la structure du tricot après tricotage par effet thermique. Cette opération de thermofixation est couramment utilisée par l'homme de l'art lors de la fabrication d'un tricot dont on veut figer la structure tridimensionnelle. Elle peut être mise en oeuvre à l'aide de différentes technologies soit par passage du tricot dans une série de fours thermorégulés, soit par passage du tricot dans un autoclave, soit encore par passage du tricot entre un ou plusieurs cylindres chauffés. Dans le cadre de la présente invention, on préférera réaliser cette opération de thermofixation par passage du tricot entre 2 cylindres chauffés.

Selon l'invention, ce tricot est réalisé à l'aide de fils continus à filaments non élastiques. Par fil continu à filaments, on entend un fil formé de un ou plusieurs filaments longs retors.

Ce fil sera de préférence choisi parmi les fils de 33 à 115 dtex comprenant 12 à 36 filaments.

Le matériau constitutif des fils est de préférence de nature synthétique et hydrophobe. Ce matériau est avantageusement choisi parmi les polyesters et les polyamides.

Selon la version préférée de l'invention, on utilisera un tricot avec fils tramés thermofixé à base de fils continus en polyester 50 dtex comprenant 24 filaments.

Ce tricot pourra présenter un grammage compris entre 20 à 40 g/m$^2$ et de préférence entre 24 à 32 g/m$^2$.

Ce tricot pourra présenter des mailles rectangulaires, carrées ou polygonales.

Ces mailles présenteront avantageusement des ouvertures dont la surface unitaire avant enrobage est de l'ordre de 0,5 à 3 mm$^2$ et de préférence comprise entre 0,85 et 1,25 mm$^2$.

Selon un mode préféré de réalisation, on utilisera un tricot qui présente des mailles trapézoïdales.

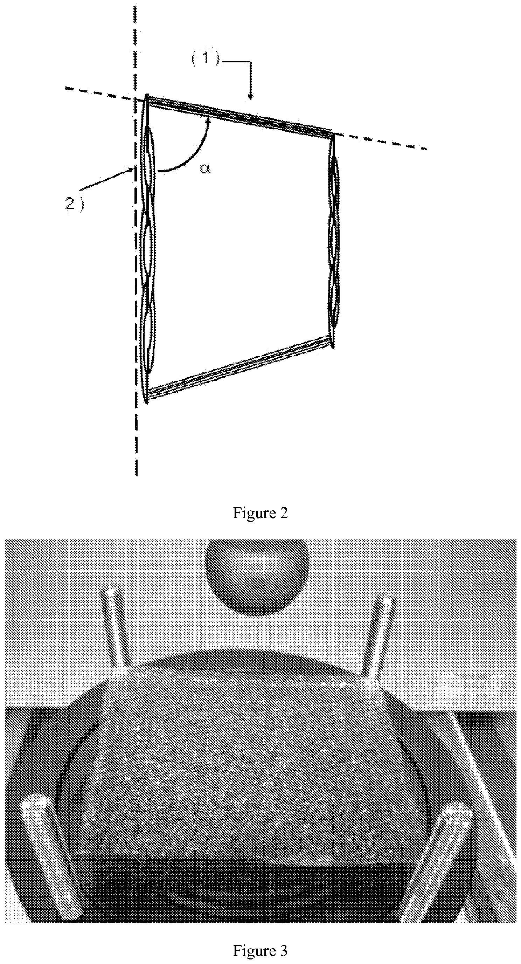

Selon une version préférée de la présente invention, on utilisera un tricot qui présente des mailles trapézoïdales. Un tricot avec une telle maille est illustré de façon schématique sur la figure 2. L'angle $\alpha$ est défini comme l'angle de raccordement moyen des rangées (1) et des colonnes (2) du tricot.

Selon la version préférée de la présente invention, ce tricot présente une surface de maille moyenne de l'ordre de 0,95 mm$^2$ et un angle $\alpha$ tel que décrit sur la figue 1 compris entre 75 et 85 degrés.

L'enduction du gel cohésif sur le tricot est réalisée de façon à laisser les mailles essentiellement non obturées, selon les techniques connues de l'homme de l'art et de manière à obtenir une enduction qui varie de 110 à 160 g/m$^2$ et de préférence de 125 à 135 g/m$^2$.

Dans le cadre de la présente invention, pour réaliser cette enduction on préfèrera mettre en oeuvre le procédé décrit dans la demande de brevet WO 00/16725.

La figure 2 annexée représente de façon schématique une maille du tissu d'un pansement selon un mode de réalisation préféré de l'invention.

Selon un second mode de réalisation alternatif, l'enveloppe constituée d'un matériau interface autoporté peut comprendre un autre type de support formé d'un tissu à maille ouverte constituée par des fils peu extensibles et peu élastiques. Ces fils sont de préférence en polyester. Il s'agit de fils multifilaments tissés de diamètre compris entre 10 et 20 $\mu$M et préférentiellement de 16 $\mu$M environ. L'ouverture des mailles est comprise entre 1 et 1.1 mm2 et de préférence de 1.05 mm2 environ. Ce support comprend de préférence un nombre de 7 fils par cm pris dans son sens longitudinal, et un nombre de 8 fils par cm pris dans son sens transversal. Le grammage de ce support est compris entre 40 et 45 g/cm2 et est de préférence de 43 g/cm2 environ. Enfin son extensibilité à 20% a été mesurée selon la norme NF EN 13726-4. Cette dernière est comprise entre 20 et 30 N/cm dans le sens longitudinal, et est préférentiellement de 24 N/cm. De la même manière, cette extensibilité dans le sens transversal est comprise entre 1 et 5 N/cm et est de préférence de 3 N/cm.

*Matériau ou ensemble de matériaux formant des canaux d'écoulement des fluides*

Le matériau composite de remplissage de plaie selon la présente invention comprend une enveloppe décrite précédemment, renfermant un matériau ou un ensemble de matériaux formant des canaux d'écoulement des fluides, plus particulièrement des exsudats des plaies.

Les matériaux introduits dans l'enveloppe du matériau composite selon l'invention peuvent être poreux ou non poreux, compressibles ou non compressibles, déformables ou non déformables, résilients ou non résilients, pour autant qu'ils remplissent leur fonction de former, intrinsèquement et/ ou par leur agencement les uns par rapport aux autres, des canaux d'écoulement des fluides.

On entend par « matériau poreux » au sens de la présente demande, tout matériau dont la structure présente des cavités pouvant former des canaux d'écoulement de fluides.

On entend par « matériau compressible » tout matériau dont le volume diminue et dont la forme est modifiée sous l'effet d'une contrainte physique externe.

On entend par « matériau déformable » tout matériau dont la forme est modifiée sous l'effet d'une contrainte physique externe mais dont le volume reste constant.

On entend par « matériau résilient » tout matériau compressible ou déformable ayant la propriété de retrouver son volume initial et/ou sa forme initiale une fois la contrainte physique externe levée. En d'autres termes, on entend par matériau résilient un matériau à mémoire de forme.

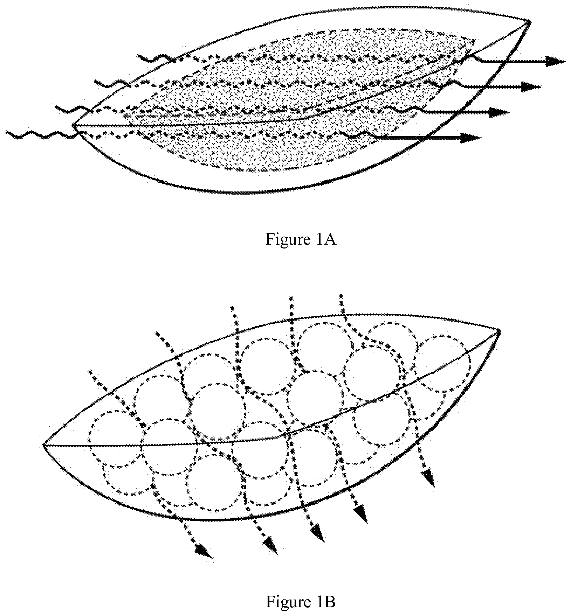

Le matériau composite de la présente invention est constitué d'une « enveloppe renfermant un matériau ou un ensemble de matériaux », c'est-à-dire qu'il se présente sous la forme d'une structure de type coeur/écorce, le coeur étant constitué par un matériau ou un ensemble de matériaux formant des canaux d'écoulement de fluide, l'écorce, appelée également enveloppe, étant constituée d'un matériau d'un matériau interface autoporté. Il est à noter que l'écorce enferme complètement le matériau ou l'ensemble de matériaux formant des canaux d'écoulement de fluide. Le ou les matériaux formant des canaux d'écoulement de fluides peuvent présenter une mobilité plus ou moins importante au sein de l'enveloppe. De telles structures sont schématisées aux figures 1 A et B.

Selon un premier mode préféré de réalisation, l'enveloppe renferme un (unique) matériau formant des canaux d'écoulement de fluides. Dans ce mode de réalisation, le matériau est poreux, compressible et résilient. La porosité du matériau lui confère la propriété de former des canaux d'écoulement des fluides de manière intrinsèque, du fait des propriétés et de la nature du matériau utilisé, présentant dans sa structure des canaux d'écoulement des fluides.

Selon ce premier mode de réalisation, l'enveloppe peut renfermer un (unique) matériau associant diverses propriétés, c'est-à-dire pouvant être poreux ou non poreux, compressible ou non compressible, déformable ou non déformable, résilient ou non résilient, pour autant qu'ils remplit sa fonction de former, intrinsèquement des canaux d'écoulement des fluides.

Ce premier mode de réalisation est visualisable à la figure 1 A dans laquelle les canaux d'écoulement de fluides laissent passer les exsudats comme illustré de manière schématique et purement illustrative par des flèches sur la figure.

Selon un second mode préféré de réalisation, l'enveloppe renferme un ensemble de matériaux formant des canaux d'écoulement des fluides.

Dans ce second mode de réalisation, les matériaux sont distincts les uns des autres à l'intérieur de l'enveloppe et séparés par un ou plusieurs interstices pouvant constituer des canaux d'écoulement de fluides.

Dans ce second mode de réalisation, les matériaux peuvent, selon un premier aspect préféré, être poreux, compressibles et résilients. Les canaux d'écoulement de fluides peuvent alors être formés à la fois par la porosité intrinsèque des matériaux constituant l'ensemble, et par les interstices présents entre les différents matériaux. Les matériaux étant compressibles et résilients, et pouvant en outre bouger les uns par rapport aux autres à l'intérieur de l'enveloppe, l'ensemble de ces matériaux est également compressible et résilient.

Alternativement, dans ce second mode de réalisation, les matériaux peuvent, selon un second aspect préféré, être non poreux, non compressibles et non déformables. Les canaux d'écoulement de fluides sont alors formés uniquement par les interstices présents entre les différents matériaux. Les canaux d'écoulement de fluides laissent passer les exsudats comme illustré de manière schématique et purement illustrative par des flèches sur la figure 1 B. Les matériaux non compressibles et non déformables peuvent bouger les uns par rapport aux autres à l'intérieur de l'enveloppe, conférant ainsi à l'ensemble de ces matériaux un caractère déformable.

Ce second mode de réalisation est visualisable à la figure 1 B.

Selon une alternative particulière, il est tout à fait envisageable de prévoir une association desdits matériaux cités précédemment. Ainsi, il est possible d'associer, selon un mode de réalisation de l'invention, des matériaux poreux ou non poreux, compressibles ou non compressibles, résilients ou non résilients et déformables ou non déformables constitutifs alors d'un ensemble de matériaux formant des canaux d'écoulement des fluides. Les propriétés intrinsèques de chaque matériau poreux, présentant, de par sa structure, des canaux d'écoulement des fluides inhérents, et les propriétés conférées par un ensemble de matériaux, poreux ou non, sont bien entendu conservées dans ce type d'association. Ainsi, des canaux d'écoulements des fluides peuvent à la fois être formés par la porosité intrinsèque d'un matériau, mais aussi par les interstices séparant différents matériaux agencés dans un ensemble, notamment si ces derniers sont aptes à bouger les uns par rapport aux autres.

Le matériau poreux, compressible et résilient de remplissage de l'enveloppe peut, par exemple, comprendre une ou plusieurs mousses, ou gazes, mais tout autre matériau approprié ayant les caractéristiques physiques requises peut être utilisé comme matériau de remplissage des enveloppes. A titre d'exemple de matériau poreux, compressible et résilient de remplissage de l'enveloppe, on peut citer des mousses polyuréthane, des mousses à base de poly(vinyl alcool), ou encore à base de cellulose, ou d'amidon, ou bien différents produits textiles, à base de fibres synthétiques ou naturelles choisies parmi la liste non limitative de composés constituée notamment du coton, du lin, de la laine, de la soie, des chlorofibres, du polyester, des polyoléfines, préférentiellement du polyéthylène, ou encore des fibres polyacrylique ou polyamide et ce sous quelque forme que ce soit, fil, fibre, tricot, tissé, non tissé, ou étoffe, etc...

Ce matériau de remplissage de l'enveloppe doit être à la fois compressible, et également suffisamment dur pour éloigner les tissus cutanés dans le lit de la plaie, sans être trop agressif pour les tissus.

Le matériau poreux de remplissage de l'enveloppe peut ainsi présenter une dureté allant de 5 à 100 Shore A et préférentiellement 20 à 100 Shore A.

Les matériaux non poreux, non compressibles et non déformables de remplissage de l'enveloppe peuvent, par exemple, être choisis parmi le PMMA (poly(methyl methacrylate)), le verre, le polystyrène, le PVC, l'acrylonitrile butadiene styrene (ABS), le silicone, le SAN (styrène acrylonitrile), le polyuréthane, l'alcool polyvinylique, la cellulose, le polyester, les polyoléfines, le polyéthylène, ou un mélange de ces matériaux. Selon un mode préféré de réalisation, les matériaux non poreux, non compressibles et non déformables de remplissage de l'enveloppe peuvent être choisis parmi les billes de verre, de polystyrène ou de silicone, ou de polyéthylène.

Actifs

Divers composés peuvent en outre être ajoutés dans l'enveloppe et/ou dans le matériau de remplissage des matériaux composites de la présente invention, comme, en particulier, des agents actifs ou des adjuvants couramment utilisés dans le domaine du traitement des plaies ou dans le domaine pharmacologique.

Le matériau composite peut contenir des principes actifs ayant un rôle favorable dans le traitement de la plaie. Ces principes actifs peuvent notamment induire ou accélérer la cicatrisation de la plaie. D'autres agents actifs peuvent également d'être utilisés dans le cadre de l'invention, comme par exemple les agents bactéricides ou bactériostatiques, les antiseptiques, les agents anti-douleurs ou les anesthésiques locaux, les agents anti-inflammatoires, les antiprurigineux, les agents apaisants, les agents hydratants, les agents anti-oxydants, les agents dépigmentants et leurs mélanges.

De manière générale, ces actifs peuvent être choisis parmi :

- les actifs favorisant la cicatrisation tels que le Rétinol, la Vitamine A, la Vitamine E, la N-acétyl-hydroxyproline, les extraits de Centella Asiatica, la papaïne, les silicones, les huiles essentielles de thym, de niaouli, de romarin et de sauge, l'acide hyaluronique, , l'Allantoïne, - Hema'tîte (gattefossé), Vitamine C, TEGO Pep 4-17(evonik), Toniskin (silab), Collageneer (Expanscience), Timecode (Seppic), Gatuline skin repair (gattefossé), Panthenol, PhytoCellTec Alp Rose (Mibelle Biochemistry), Erasyal(libragen), Serilesine (Lipotec), Heterosides de Talapetraka (beyer), Stoechiol(codif), macarose (Sensient), Dermaveil (Ichimaru Pharcos), Phycosaccaride AI (Codif), les facteurs de croissance, la metformine, les oligosaccharides polysulfatés synthétiques ayant 1 à 4 unités oses tels qu'en particulier le sel de potassium du sucrose octasulfate (connu sous l'abréviation KSOS), commercialisé dans le produit Urgotul® Start par les Laboratoires URGO;

- les agents bactéricides ou bactériostatiques tels que le Polymyxine B, les pénicillines (Amoxycilline), l'acide clavulanique, les tétracyclines, la Minocycline, la chlorotétracycline, les aminoglycosides, l'Amikacine, la Gentamicine, la Néomycine, les probiotiques, sels d'argent tels que par exemple le sulfate d'argent, le chlorure d'argent, le nitrate d'argent, la sulfadiazine argentique, des ammoniums quaternaires, le polyhexaméthylène biguanide et la Chlorhexidine;

- les antiseptiques tels que le mercurothiolate de sodium, l'éosine, la chlorhexidine, le borate de phénylmercure, l'eau oxygénée, la liqueur de Dakin, le triclosan, le biguanide, l'hexamidine, le thymol, le Lugol, la Povidone iodée, le Merbromine, le Chlorure de Benzalkonium et de Benzethonium, l'éthanol, l'isopropanol ;

- les anti-douleurs ou les anesthésiques locaux tels que le Paracétamol, la Codéine, le Dextropropoxyphène, le Tramadol, la Morphine et ses dérivés, les Corticoïdes et dérivés ;

- les anti-inflammatoires tels que les Glucocorticoïdes, les anti-inflammatoires non stéroïdiens, l'Aspirine, l'Ibuprofène, le Kétoprofène, le Flurbiprofène, le Diclofénac, l'Acéclofénac, le Kétorolac, le Méloxicam, le Piroxicam, le Ténoxicam, le Naproxène, l'Indométacine, le Naproxcinod, le Nimésulide, le Célécoxib, l'Etoricoxib, le Parécoxib, le Rofécoxib, le Valdécoxib, la Phénylbutazone, l'acide niflumique, l'acide méfénamique ;

- les agents dépigmentants tels que l'acide kojique (Kojic Acid SL® - Quimasso (Sino Lion)), l'Arbutine (Olevatin® - Quimasso (Sino Lion)), le mélange de palmitoylpropyl de sodium et d'extrait de nénuphar blanc (Sepicalm® - Seppic), l'undécylénoyl phénylalanine (Sepiwhite® - Seppic),

- les antiprurigineux : hydrocotisone, enoxolone, diphenyhydramine, antihistaminique à application locale anti H1

- les actifs hydratants tels que xpermoist (lipotec), Acide hyaluronique, Urée, acides gras, Glycérine, Cires, Exossine(unipex)

- les filtres UV tels que Parsol MCX, Parsol 1789

- les agents apaisants tels que de la camomille, du bisabolol, du xanthalène, de l'acide glycyrrhébénique, tanactine (CPN), Calmiskin (Silab),

- les agents anti-oxydants, tels que la vitamine E.

Selon un mode préféré de réalisation, les actifs pouvant être introduits dans l'enveloppe et/ou dans le matériau de remplissage des matériaux composites selon la présente invention sont de préférence choisis parmi les actifs favorisant la cicatrisation, les anti-inflammatoires et leur mélange.

Par « actif favorisant la cicatrisation », on entend tout actif capable d'intervenir favorablement à quelconque étape du processus cicatriciel et via n'importe quel type d'interaction que ce soit, c'est-à-dire par toute interaction de nature biologique, chimique ou physique avec la plaie au contact de laquelle ledit actif se trouve dispensé.

Plus particulièrement, les actifs pouvant être introduits dans l'enveloppe et/ou dans le matériau de remplissage des matériaux composites selon la présente invention sont de préférence choisis parmi la metformine, les oligosaccharides polysulfatés synthétiques ayant 1 à 4 unités oses tels qu'en particulier le sel de potassium du sucrose octasulfate, l'aspirine, le sulfate d'argent, la sulfadiazine argentique et leurs mélanges.

D'une façon générale, les matériaux composites selon la présente invention peuvent comprendre des agents actifs dans l'enveloppe et/ou dans le matériau de remplissage en une quantité de 0,01 à 20 % en poids, de préférence de 1 à 15 % en poids et de préférence encore de 2 à 10 % en poids, rapportée au poids total de l'enveloppe et/ou du matériau de remplissage les contenant.

Comme adjuvants, on peut citer des matières colorantes, des charges, des absorbeurs ou piégeurs d'odeurs, des régulateurs de pH, des microcapsules ou des microsphères qui peuvent éventuellement contenir des agents actifs, de la vaseline, des polymères ou des tensioactifs permettant d'optimiser la vitesse de gélification, de mouillabilité ou le relargage des actifs du matériau composite.

Le matériau composite de remplissage des plaies selon l'invention peut se présenter sous toute forme géométrique souhaitée, notamment adaptée à la forme et à la profondeur de la plaie.

L'enveloppe est de préférence fermée autour du matériau ou un ensemble de matériaux de remplissage formant des canaux d'écoulement des fluides par thermoscellage, par couture ou par un ou plusieurs noeuds au niveau de l'enveloppe, préférentiellement par thermoscellage

Selon un mode particulier de réalisation, un ou plusieurs matériaux poreux, compressibles et élastiques de remplissage peuvent être introduits dans une même enveloppe.

Selon un mode particulier de réalisation, l'ensemble de matériaux formant des canaux d'écoulement des fluides peut se présenter sous la forme d'un « collier de perles », c'est-à-dire que plusieurs matériaux de remplissage formant des canaux d'écoulement des fluides peuvent être répartis individuellement dans autant de cavités de l'enveloppe non tissée, séparées entre elles par thermoscellage, par couture ou par un ou plusieurs noeuds au niveau de l'enveloppe, préférentiellement par thermoscellage de ladite enveloppe.

La présente invention est illustrée plus en détails dans l'exemple non limitatif suivant.

<u>EXEMPLE</u>

**Objectif** :

On a testé la résistance mécanique (notamment à la déstructuration) de différents matériaux composites de remplissage de plaies dans des conditions proches des TPN (c'est à dire sous vide à 125 mmHg), afin d'observer, parmi les différents matériaux testés, lesquels résistent à la force exercée et lesquels se rompent.
On a utilisé les appareils et solution suivants :

- un dynamomètre MECA - 004/SYN200, capable d'appliquer une force de 26 N au contact de la matière souhaitée

- un capteur 100N/MECA-008 attenant au dynamomètre

- une tige métallique terminée par une bille d'acier poli, de diamètre 25,3866 mm et de circularité au niveau de l'équateur de 0,0093 mm.

- une pince (de serrage) annulaire, d'un diamètre intérieur de 44.4763 mm.

- une solution NaCl/CaCl$_2$ comprenant du NaCl (8.298 g +/-5%) et du CaCl$_2$ (0.368g +/-5%)]

On a testé un matériau interface autoporté formé d'une couche mince d'une composition comprenant une matrice hydrophobe et comportant des trous traversants, ladite matrice hydrophobe comprenant :

- pour 100 parties en poids d'un copolymère tribloc styrène-oléfine saturée-styrène présentant une viscosité comprise entre 0,2 et 2 Pa.s, telle que mesurée dans une solution à 10 % (masse/masse) dans le toluène ;

- de 400 à 1 220 parties en poids d'un plastifiant, de préférence une huile plastifiante ; et

- de 0 à 720 parties en poids de vaseline ;

étant en outre précisé que :

- la quantité totale de plastifiant et de vaseline est supérieure ou égale à 750 parties en poids ;

la quantité de vaseline est comprise entre 400 et 720 parties en poids lorsque la quantité de plastifiant est comprise entre 1 000 et 1 220 parties en poids, ledit matériau interface constituant une enveloppe possible du matériau composite de remplissage d'une plaie selon l'invention.
On a utilisé comme matériau formant des canaux d'écoulement des fluides, une mousse polyuréthane réticulée hydrophobe commercialisée par la société AQF® sous la référence commerciale PDQZ30.

**Mode opératoire**

• <u>Force à appliquer aux échantillons</u>

Les systèmes TPN sont réglés de façon la plus courante pour exercer un vide à 125mmHg. Ceci correspond à exercer une force de 26 N sur le matériau composite de remplissage de la plaie.

• <u>Préparation des échantillons</u>

On a découpé à l'aide d'un emporte-pièce 3 échantillons carrés de 80 mm de côté de la mousse AQF PDQZ30 constituant le matériau de remplissage dans l'enveloppe.
On a également découpé à l'aide d'un emporte-pièce 1 échantillon carré de 80 mm de côté du matériau interface autoporté constituant l'enveloppe à tester.
On prépare en parallèle une solution d'essai comprenant du NaCl (8.298 g +/-5%) et du CaCl$_2$ (0.368g +-/5%)]. Cette solution permet, d'une part, de simuler les conditions d'humidité retrouvées au sein d'une plaie et, d'autre part, de simuler la concentration saline des exsudats retrouvés au niveau d'une plaie.
Enfin, on trempe le carré de matériau interface autoporté constituant l'enveloppe à tester dans la solution d'essai pendant 30minutes à 37°C + 2°C. Après 30minutes, on retire le carré et on le laisse égoutter pendant environ 30 secondes.

On superpose ensuite le carré de matériau interface autoporté hydraté sur le carré de mousse (qui elle est non hydratée) puis on positionne le matériau composite ainsi obtenu dans le dispositif de test. La figure 3 illustre le dispositif de test sur lequel on a déposé l'échantillon de matériau composite constitué du carré de mousse et du matériau interface autoporté superposés.

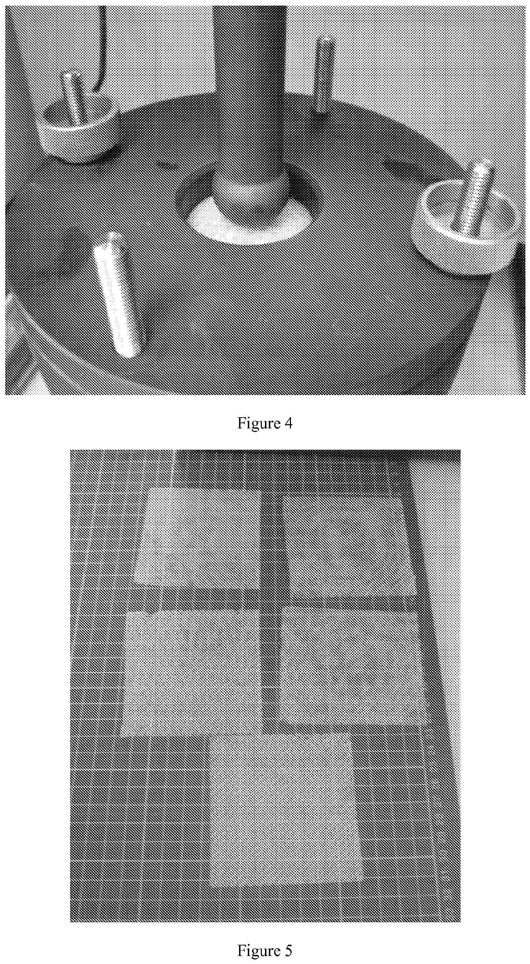

Une pince de serrage annulaire est ensuite apposée sur l'échantillon constitué du matériau composite et est fixée au moyen de 4 vis de serrage de manière à assurer la complète cohésion entre les deux matériaux. La figure 4 illustre une photo du dispositif après mise en place de la pince de serrage.

On règle le dynamomètre de sorte à ce que la vitesse de descente verticale de la tige métallique terminée par la bille corresponde à (300 + 10) mm/min et de manière à ce que cette descente soit stoppée lorsque la tige métallique terminée par la bille exerce une force de 26N après être arrivée au contact de l'échantillon. Après contact et exercice de la pression souhaitée avec l'échantillon, la tige métallique terminée par la bille s'arrête et remonte.

On observe ensuite la résistance de l'échantillon à ce premier cycle.

Si l'échantillon a résisté à ce premier cycle, on reproduit 5 fois le cycle de « montée et descente » de la tige métallique terminée par la bille sur l'échantillon, ladite bille exerçant, à chaque cycle, une force de 26 N sur l'échantillon.

### Résultats :

Le tableau ci-après résume l'état de l'échantillon de matériau composite après 1 et 5 cycles de test :

| Produit testés | Résultats |
|---|---|
| Echantillon mettant en oeuvre le matériau interface autoporté selon l'invention (1 cycle) | Pas de rupture du produit |
| Echantillon mettant en oeuvre le matériau interface autoporté selon l'invention (5 cycles) | Pas de rupture du produit |

La figure 5 illustre l'échantillon mettant en oeuvre le matériau interface autoporté selon l'invention après 5 cycles de test. On remarque que le produit ne subit que de très légères déformations, et en aucun cas ne se destructure ni totalement, ni partiellement.

Ledit matériau interface autoporté selon l'invention est donc un nouveau matériau assimilable à un gel non adhérent pouvant être utilisé seul, c'est-à-dire, sans nécessité d'un support pour le soutenir comme cela est décrit dans les demandes WO2009/071928 et WO2009/071938. Lorsqu'il est testé sous forme d'un matériau composite de remplissage d'une plaie comme dans le présent exemple, il présente une bonne résistance à la pression dans des conditions asssimilables à celles exercées en TPN.

### Revendications

1. Matériau composite de remplissage de plaie comprenant une enveloppe renfermant un matériau ou un ensemble de matériaux formant des canaux d'écoulement de fluide, ladite enveloppe étant constituée d'un matériau interface autoporté formé d'une couche mince d'une composition comprenant une matrice hydrophobe et comportant des trous traversants, ladite matrice hydrophobe comprenant :

   - pour 100 parties en poids d'un copolymère tribloc styrène-oléfine saturée-styrène présentant une viscosité comprise entre 0,2 et 2 Pa.s, telle que mesurée dans une solution à 10 % (masse/masse) dans le toluène ;
   - de 400 à 1 220 parties en poids d'un plastifiant, de préférence une huile plastifiante ; et
   - de 0 à 720 parties en poids de vaseline ;

   étant en outre précisé que :

   - la quantité totale de plastifiant et de vaseline est supérieure ou égale à 750 parties en poids ;
   - la quantité de vaseline est comprise entre 400 et 720 parties en poids lorsque la quantité de plastifiant est comprise entre 1 000 et 1 220 parties en poids.

2. Matériau composite de remplissage selon la revendication 1, **caractérisé en ce que** la matrice hydrophobe précitée comprend, pour 100 parties en poids du copolymère précité :

- de 600 à 900 parties en poids d'un plastifiant, de préférence une huile plastifiante ; et
- de 150 à 450 parties en poids de vaseline.

**3.** Matériau composite de remplissage selon l'une quelconque des revendications précédentes, **caractérisé en ce que** la composition précitée comprenant une matrice hydrophobe comprend, en outre, des particules d'hydrocolloïde en une quantité inférieure ou égale à 25 % en poids rapportée au poids total de ladite matrice hydrophobe.

**4.** Matériau composite de remplissage selon l'une des revendications précédentes, **caractérisé en ce que** l'enveloppe en matériau autoporté se présente sous la forme d'un filet aéré ou grille présentant une épaisseur comprise entre 0,4 et 2 mm, de préférence entre 0,5 mm et 1 mm , de préférence encore de l'ordre de 0,6 mm.

**5.** Matériau composite de remplissage selon l'une des revendications précédentes, **caractérisé en ce que** les trous traversants du matériau interface autoporté sont de géométrie quelconque et présenteront par exemple une section transversale circulaire, rectangulaire, trapézoïdale ou carrée, présentent surface est comprise entre 0,25 et 5 mm$^2$ ; la surface totale desdits trous représentant entre 20 et 70 %, de préférence entre 30 et 50 % de la surface totale dudit matériau interface autoporté.

**6.** Matériau composite de remplissage de plaie selon l'une quelconque des revendications précédentes, **caractérisé en ce que** l'enveloppe comprend un support formé d'un tissu de fils en matériau flexible et très peu extensible et non élastique, les fils constituant le tissu sont de nature hydrophobe, de préférence en polyester, notamment du polyéthylène téréphtalate.

**7.** Matériau composite de remplissage de plaie selon l'une quelconque des revendications précédentes, **caractérisé en ce que** les matériaux introduits dans l'enveloppe peuvent être poreux ou non poreux, compressibles ou non compressibles, déformables ou non déformables, résilients ou non résilients, pour autant qu'ils remplissent leur fonction de former des canaux d'écoulement de fluides.

**8.** Matériau composite de remplissage de plaie selon l'une quelconque des revendications précédentes, **caractérisé en ce que** l'enveloppe renferme un matériau formant des canaux d'écoulement de fluides, ledit matériau étant poreux, compressible et résilient.

**9.** Matériau composite de remplissage de plaie selon l'une quelconque des revendications 1 à 7, **caractérisé en ce que** l'enveloppe renferme un ensemble de matériaux formant des canaux d'écoulement de fluides.

**10.** Matériau composite de remplissage de plaie selon la revendication 9, **caractérisé en ce que** les matériaux sont poreux, compressibles et résilients.

**11.** Matériau composite de remplissage de plaie selon la revendication 9, **caractérisé en ce que** les matériaux sont non poreux, non compressibles, et non déformables.

**12.** Matériau composite de remplissage de plaie selon l'une quelconque des revendications 7 à 10, **caractérisé en ce que** le ou les matériaux poreux, compressibles et résilients de remplissage de l'enveloppe comprennent une ou plusieurs mousses, ou gazes, et/ou présentent une dureté allant de 5 à 100 Shore A et préférentiellement de 20 à 100 Shore A.

**Patentansprüche**

**1.** Verbundmaterial zur Wundfüllung, umfassend eine Hülle, welche ein Material oder eine Materialgruppe umschließt, die Kanäle für einen Fluidstrom bildet, wobei die Hülle aus einen selbsttragenden Zwischenmaterial besteht, das aus einer dünnen Schicht einer Zusammensetzung gebildet ist, die eine hydrophobe Matrix aufweist und Durchgangslöcher umfasst, wobei die hydrophobe Matrix umfasst:

- auf 100 Gewichtsteile eines Styrol-gesättigten Styrol-Olefin Triblockcopolymers mit einer Viskosität von 0,2 bis 2 Pa.s, gemessen in einer Lösung mit 10 % (Masse/Masse) in Toluol;
- 400 bis 1 220 Gewichtsteile eines Weichmachers, bevorzugt eines Weichmacheröls; und
- 0 bis 720 Gewichtsteile Vaseline;

wobei ferner präzisiert wird, dass

- die Gesamtmenge Weichmacher und Vaseline größer gleich 750 Gewichtsteile ist;
- die Vaseline-Menge 400 bis 720 Gewichtsteile beträgt, sofern die Weichmachermenge 1 000 bis 1 220 Gewichtsteile beträgt.

2. Füll-Verbundmaterial nach Anspruch 1, **dadurch gekennzeichnet, dass** die oben genannte hydrophobe Matrix auf 100 Gewichtsteile des oben genannten Copolymers umfasst:

- 600 bis 900 Gewichtsteile eines Weichmachers, bevorzugt Weichmacheröl; und
- 150 bis 450 Gewichtsteile Vaseline.

3. Füll-Verbundmaterial nach einem der vorhergehenden Ansprüche, **dadurch gekennzeichnet, dass** die oben genannte, eine hydrophobe Matrix umfassende Zusammensetzung ferner Hydrokolloidpartikel in einer Menge kleiner gleich 25 Gewichtsprozent bezogen auf das Gesamtgewicht der hydrophoben Matrix umfasst.

4. Füll-Verbundmaterial nach einem der vorhergehenden Ansprüche, **dadurch gekennzeichnet, dass** die Hülle aus selbsttragendem Material in Form eines luftigen Netzes oder eines Gitters mit einer Dicke von 0,4 bis 2 mm, bevorzugt von 0,5 bis 1 mm, noch stärker bevorzugt von ca. 0,6 mm vorliegt.

5. Füll-Verbundmaterial nach einem der vorhergehenden Ansprüche, **dadurch gekennzeichnet, dass** die Durchgangslöcher des selbsttragenden Zwischenmaterials eine beliebige Geometrie aufweisen und beispielsweise einen kreisrunden, rechteckigen, trapezförmigen oder quadratischen Querschnitt aufweisen und eine Fläche von 0,25 bis 5 mm$^2$ aufweisen; wobei die Gesamtfläche der Löcher 20 bis 70 %, bevorzugt 30 bis 50 % der Gesamtfläche des selbsttragenden Zwischenmaterials ausmacht.

6. Verbundmaterial zur Wundfüllung nach einem der vorhergehenden Ansprüche, **dadurch gekennzeichnet, dass** die Hülle eine Stütze umfasst, die aus einem Gewebe aus Fäden aus flexiblem, sehr wenig dehnbarem und nicht elastischem Material gebildet ist, wobei die das Gewebe bildenden Fäden hydrophob sind, vorzugsweise aus Polyester und insbesondere aus Polyethylenterephtalat.

7. Verbundmaterial zur Wundfüllung nach einem der vorhergehenden Ansprüche, **dadurch gekennzeichnet, dass** die in die Hülle eingeführten Materialien porös oder nicht porös, komprimierbar oder nicht komprimierbar, verformbar oder nicht verformbar, elastisch oder nicht elastisch sein können, solange sie ihre Funktion, Kanäle für einen Fluidstrom zu bilden, erfüllen.

8. Verbundmaterial zur Wundfüllung nach einem der vorhergehenden Ansprüche, **dadurch gekennzeichnet, dass** die Hülle ein Material einschließt, welches Kanäle für einen Fluidstrom bildet, wobei das Material porös, komprimierbar und elastisch ist.

9. Verbundmaterial zur Wundfüllung nach einem der Ansprüche 1 bis 7, **dadurch gekennzeichnet, dass** die Hülle eine Gruppe von Materialien umschließt, welche Kanäle für einen Fluidstrom bilden.

10. Verbundmaterial zur Wundfüllung nach Anspruch 9, **dadurch gekennzeichnet, dass** die Materialien porös, komprimierbar und elastisch sind.

11. Verbundmaterial zur Wundfüllung nach Anspruch 9, **dadurch gekennzeichnet, dass** die Materialien nicht porös, nicht komprimierbar und nicht verformbar sind.

12. Verbundmaterial zur Wundfüllung nach einem der Ansprüche 7 bis 10, **dadurch gekennzeichnet, dass** das oder die porösen, komprimierbaren und elastischen Füllmaterialien der Hülle einen oder mehrere Schäume oder Gaze umfassen und/oder eine Härte von 5 bis 100 Shore A und bevorzugt von 20 bis 100 Shore A aufweisen.

**Claims**

1. A composite wound packing material comprising a casing enclosing a material, or an assembly of materials, forming fluid flow channels, said casing consisting of a self-supporting interface material formed from a thin layer of a composition comprising a hydrophobic matrix and comprising through-holes, said hydrophobic matrix comprising:

- per 100 parts by weight of a styrene/saturated olefin/styrene triblock copolymer having a viscosity of between 0.2 and 2 Pa.s, as measured in a 10% (weight/weight) solution in toluene;
- from 400 to 1220 parts by weight of a plasticizer, preferably a plasticizing oil; and
- from 0 to 720 parts by weight of petroleum jelly;

it being specified, moreover, that:

- the total amount of plasticizer and petroleum jelly is greater than or equal to 750 parts by weight;
- the amount of petroleum jelly is between 400 and 720 parts by weight when the amount of plasticizer is between 1000 and 1220 parts by weight.

2. The composite wound packing material as claimed in claim 1, **characterized in that** the abovementioned hydrophobic matrix comprises, per 100 parts by weight of the abovementioned copolymer:

- from 600 to 900 parts by weight of a plasticizer, preferably a plasticizing oil; and
- from 150 to 450 parts by weight of petroleum jelly.

3. Composite wound packing material as claimed in either one of the preceding claims, **characterized in that** the abovementioned composition comprising a hydrophobic matrix also comprises hydrocolloid particles at an amount of less than or equal to 25% by weight relative to the total weight of said hydrophobic matrix.

4. Composite wound packing material as claimed in one of the preceding claims, **characterized in that** the casing made of self-supporting material is in the form of a breathable net or grid having a thickness of between 0.4 and 2 mm, preferably between 0.5 and 1 mm, more preferably still of the order of 0.6 mm.

5. Composite wound packing material as claimed in one of the preceding claims, **characterized in that** the through-holes of the self-supporting interface material have any geometry and will have, for example, a circular, rectangular, trapezoidal or square cross section, have a surface area between 0.25 and 5 mm$^2$; the total surface area of said holes representing between 20 and 70%, preferably between 30 and 50% of the total surface area of said self-supporting interface material.

6. Composite wound packing material as claimed in any one of the preceding claims, **characterized in that** the casing comprises a support formed from a fabric of yarns made of flexible, barely extensible and non-elastic material; the yarns constituting the fabric are hydrophobic in nature and preferably made from polyester, especially polyethylene terephthalate.

7. The composite wound packing material as claimed in any one of the preceding claims, **characterized in that** the materials introduced into the casing may be porous or non-porous, compressible or non-compressible, deformable or non-deformable, resilient or non-resilient, as long as they perform their function of forming fluid flow channels.

8. The composite wound packing material as claimed in any one of the preceding claims, **characterized in that** the casing encloses a material forming fluid flow channels, said material being porous, compressible and resilient.

9. The composite wound packing material as claimed in any one of claims 1 to 7, **characterized in that** the casing encloses an assembly of materials forming fluid flow channels.

10. The composite wound packing material as claimed in claim 9, **characterized in that** the materials are porous, compressible and resilient.

11. The composite wound packing material as claimed in claim 9, **characterized in that** the materials are non-porous, non-compressible and non-deformable.

12. The composite wound packing material as claimed in any one of claims 7 to 10, **characterized in that** the porous, compressible and resilient material(s) for filling the casing comprise one or more foams or gauzes, and/or have a hardness ranging from 5 to 100 Shore A and preferentially from 20 to 100 Shore A.

Figure 1A

Figure 1B

Figure 2

Figure 3

Figure 4

Figure 5

## RÉFÉRENCES CITÉES DANS LA DESCRIPTION

**Documents brevets cités dans la description**

- WO 2009071928 A
- WO 2009071938 A, Smith and Nephew
- WO 2006052839 A
- FR 2936158
- EP 1143895 A
- EP 2524706 A
- EP 2524705 A
- WO 0016725 A

**Littérature non-brevet citée dans la description**

- **HERBERT WEISS.** Coating and Laminating. *Converting Technology Machine,* 1977, 153